# FASCICULE DE BREVET EUROPEEN

(11) **EP 3 223 721 B1**
(45) Date de publication et mention de la délivrance du brevet: **29.04.2020**
(21) Numéro de dépôt: 15801783.0
(22) Date de dépôt: 26.11.2015
(51) Int. Cl.: A61B 17/122, A61B 17/128

(54) **DISPOSITIF DE TRAITEMENT D'UN TISSU CORPOREL ET NÉCESSAIRE DE TRAITEMENT ASSOCIÉ**
VORRICHTUNG ZUR BEHANDLUNG EINES KÖRPERGEWEBES UND ZUGEHÖRIGES BEHANDLUNGSKIT
DEVICE FOR TREATING A BODY TISSUE, AND ASSOCIATED TREATMENT KIT

(30) Priorité: 26.11.2014 FR 1461483
(43) Date de publication de la demande: 04.10.2017
(73) Titulaire: Heartworks LLC, Wilmington, DE 19801 (US)
(72) Inventeur: LADJALI, Mustapha, 92500 Rueil Malmaison (FR)
(74) Mandataire: Lavoix
(86) Numéro de dépôt international: PCT/EP2015/077783
(87) Numéro de publication internationale: WO 2016/083511

(56) Documents cités:
- EP-A1- 0 908 141
- EP-A2- 2 308 378
- WO-A1-99/55237
- WO-A1-2012/068002
- WO-A1-2014/111531
- WO-A2-03/092473
- US-A- 5 928 264
- US-A1- 2013 267 969

## Description

La présente invention concerne un dispositif de traitement d'un tissu corporel présentant un prolapsus, selon le préambule de la revendication 1.

Ce dispositif s'applique notamment au traitement des valves cardiaques, et en particulier au traitement des valves mitrales.

Une valve mitrale est typiquement constituée de deux feuillets, antérieur et postérieur, et contrôle le flux sanguin qui s'écoule de l'oreillette gauche vers le ventricule gauche du cœur. La valve mitrale assure une circulation univoque du flux sanguin, évitant un reflux sanguin à l'issue de la contraction ventriculaire.

Pour éviter le reflux sanguin, les feuillets de la valve doivent assurer une coaptation par leurs bords libres.

En cas de prolapsus, c'est-à-dire de relâchement de la paroi du feuillet, l'un des feuillets présente un renflement qui définit une zone en saillie épaissie et augmentée en surface. Le prolapsus peut alors empêcher la valve de se refermer de manière étanche. Il en résulte une insuffisance mitrale, c'est-à-dire un reflux du sang dans l'oreillette gauche quand le ventricule se contracte.

Un traitement possible de cette maladie consiste en une ablation chirurgicale du prolapsus. Toutefois, une telle opération invasive est fastidieuse et présente des risques non négligeables pour le patient, notamment lorsque le patient est âgé et/ou en mauvaise santé.

Pour pallier ce problème, un autre moyen connu de traiter cette maladie de manière endovasculaire consiste à placer un clip destiné à former un point de liaison entre le feuillet antérieur et le feuillet postérieur, au niveau du prolapsus. Ceci permet de rétablir la coaptation entre les feuillets de la valve au niveau du prolapsus.

Cependant, le clip forme un point de liaison permanent entre les feuillets de la valve. Ainsi, lors de l'ouverture de la valve, les feuillets s'ouvrent uniquement de part et d'autre du point de liaison créant deux ouvertures de passage distinctes. Le sang s'écoule alors par deux voies séparées l'une de l'autre par le point de liaison.

Ce type de traitement supprime le problème au niveau du prolapsus. Il ne donne cependant pas entière satisfaction, puisqu'il perturbe la coaptation des feuillets à l'écart du prolapsus, et puisqu'il perturbe le passage du sang au niveau de la valve.

Les documents WO 99/55237 A1, US 2013/267969 A1, US 5 928 264 A, WO 2012/068002 A1, EP 2 308 378 A2 et EP 0 908 141 A1 décrivent des dispositifs de traitement de tissus corporels.

Un but de l'invention est donc d'obtenir un dispositif de traitement d'un tissu présentant un prolapsus permettant de traiter le prolapsus de manière peu invasive, en perturbant le moins possible le tissu, une fois le dispositif mis en place.

En particulier, lorsque le tissu est un feuillet de valve cardiaque, un but de l'invention est de traiter le prolapsus en permettant à la valve de se refermer de manière étanche lorsque le ventricule se contracte, et en assurant une coaptation satisfaisante des feuillets.

A cet effet, l'invention a pour objet un dispositif selon la revendication 1.

Le dispositif de traitement selon l'invention peut comprendre l'une ou plusieurs des caractéristiques des revendications 1 à 6, prise(s) isolément ou suivant toute combinaison techniquement possible.

L'invention a également pour objet un nécessaire de traitement d'un tissu corporel présentant un prolapsus selon la revendication 7.

Le nécessaire de traitement selon l'invention peut comprendre la caractéristique de la revendication 8.

Il est également décrit un procédé de traitement d'un tissu corporel présentant un prolapsus, comprenant les étapes de :
- fourniture d'un nécessaire de traitement tel que défini plus haut;
- positionnement des extrémités distales de saisie des bras de pincement autour du prolapsus, les bras étant dans leur position angulaire d'accroche ;
- rotation d'un premier bras de pincement vers une position angulaire d'enserrement du prolapsus ;
- rotation du deuxième bras de pincement vers une position angulaire d'enserrement du prolapsus.

Le procédé de traitement peut en outre comprendre l'une ou plusieurs des caractéristiques suivantes, prise(s) isolément ou suivant toute combinaison techniquement possible :
- le tissu corporel est un feuillet de valve, avantageusement un feuillet de valve mitrale.
- le deuxième bras se fixe sur le premier bras dans la position angulaire d'enserrement.

L'invention sera mieux comprise à la lecture de la description qui va suivre, donnée uniquement à titre d'exemple et faite en se référant aux dessins annexés sur lesquels :
- la figure 1 est une représentation schématique d'une valve cardiaque présentant un prolapsus ;
- la figure 2 est une représentation schématique d'un nécessaire de traitement d'une valve cardiaque, comprenant un premier dispositif de traitement selon l'invention ;
- la figure 3 est une représentation schématique d'une extrémité du premier dispositif de traitement de la figure 2;
- la figure 4 est une représentation schématique d'une étape de fonctionnement du premier dispositif de traitement selon l'invention ;
- la figure 5 est une représentation schématique d'une étape ultérieure de fonctionnement du premier dispositif de traitement selon l'invention ; et
- la figure 6 est une représentation schématique d'une étape ultérieure de fonctionnement du premier dispositif de traitement selon l'invention ;
- la figure 7 est une représentation schématique d'un feuillet présentant un prolapsus, pincé et écrasé par le premier dispositif de traitement selon l'invention ;
- la figure 8 est une représentation schématique d'une extrémité du premier dispositif de traitement selon une variante de l'invention,
- la figure 9 est une représentation schématique d'un deuxième dispositif de traitement selon l'invention ;
- la figure 10 est une représentation schématique d'un troisième dispositif de traitement selon l'invention ;
- la figure 11 est une représentation schématique d'un quatrième dispositif de traitement selon l'invention ;
- la figure 12 est une représentation schématique d'un cinquième dispositif selon l'invention dans une configuration initiale de verrouillage ;
- la figure 13 est une représentation du cinquième dispositif dans une configuration libre ;
- la figure 14 est une représentation du cinquième dispositif en cours de rotation ;
- la figure 15 est une représentation du cinquième dispositif en fin de rotation ;
- la figure 16 est une représentation du cinquième dispositif en configuration de maintien ;
- la figure 17 est une représentation schématique d'un sixième dispositif de traitement selon l'invention ;
- la figure 18 est une représentation schématique du sixième dispositif de traitement selon l'invention dans une configuration de maintien ;
- la figure 19 est une représentation schématique d'un deuxième dispositif de maintien ;
- la figure 20 est une représentation schématique d'un troisième dispositif de maintien ; et
- la figure 21 est une représentation schématique d'un quatrième dispositif de maintien ;
- la figure 22 est une représentation schématique d'un septième dispositif de traitement dans une configuration libre ;
- la figure 23 est une représentation schématique du septième dispositif de traitement en cours de rotation ;
- la figure 24 est une représentation schématique du septième dispositif de traitement dans une configuration de maintien ;
- la figure 25 est une représentation schématique d'un huitième dispositif de traitement en cours de rotation ;
- la figure 26 est une représentation schématique du huitième dispositif de traitement dans une configuration de maintien.

Dans tout ce qui suit, les termes « proximal » et « distal » s'entendent respectivement comme relativement plus près de l'opérateur, et comme relativement plus éloigné de l'opérateur lors de l'utilisation du dispositif.

Dans tout ce qui suit, on entend par directions « sensiblement parallèles », que les directions forment entre elles un angle inférieur à 5°.

Dans tout ce qui suit, la direction « longitudinal » est la direction principale du tuteur. Le terme « transversal » s'entend comme dans un plan perpendiculaire à la direction longitudinal.

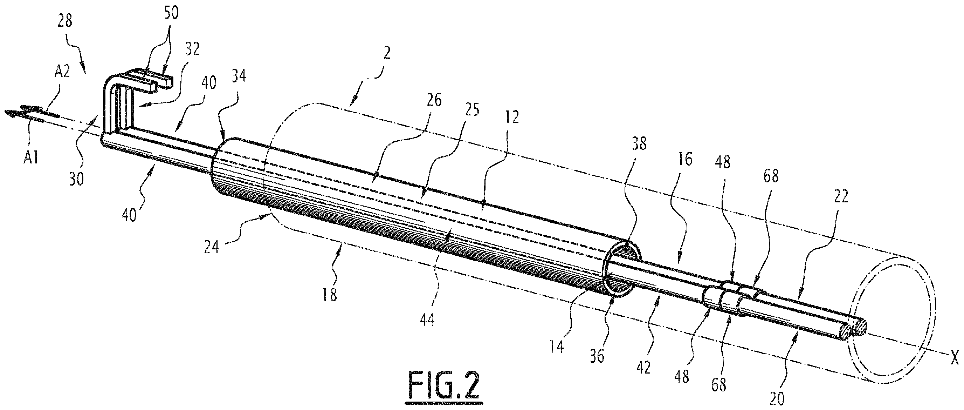

Un premier nécessaire de traitement 2 selon l'invention est représenté schématiquement sur la figure 2.

Ce premier nécessaire de traitement 2 est destiné au traitement d'un tissu corporel présentant un prolapsus 7, notamment au traitement d'une valve 4 cardiaque native telle qu'une valve mitrale.

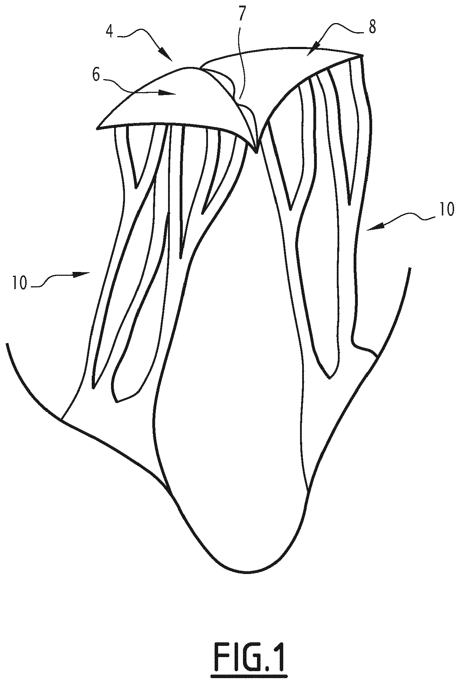

Une telle valve 4 est représentée sur la figure 1. Elle est typiquement formée par un feuillet antérieur 6 et un feuillet postérieur 8. Le prolapsus 7 est situé sur le feuillet postérieur 8. Les feuillets de la valve 4 sont reliés à la paroi du ventricule gauche par l'intermédiaire de cordages 10.

Le prolapsus 7 forme un renflement qui fait saillie au niveau du bord libre du feuillet 8 empêchant la coaptation entre les bords libres des feuillets 6, 8 de la valve 4.

Le prolapsus 7 présente, par exemple, une surface inférieure dirigée vers les cordages 10 et une surface supérieure opposée à la surface inférieure.

Comme illustré sur la figure 2, le premier nécessaire de traitement 2 selon l'invention comprend un premier dispositif de traitement 12, comportant deux bras de pincement 14, 16, et un organe de guidage 25. Le premier dispositif de traitement 12 étant propre à être implanté sur la valve 4. Le premier nécessaire de traitement 2 comporte, en outre, un tuteur 18 propre à retenir le premier dispositif 12 lors de sa mise en place, et deux mécanismes d'entrainement 20, 22, chacun étant destiné à être associé respectivement à un des deux bras de pincement 14, 16. Les deux bras de pincement 14, 16 sont guidés transversalement par l'organe de guidage 25 qui limite leur déplacement transversal l'un par rapport à l'autre.

Le tuteur 18 s'étend suivant un axe longitudinal X et définit au moins une ouverture distale 24 de retenue du dispositif 12. L'axe longitudinal X s'étend suivant une direction principale X.

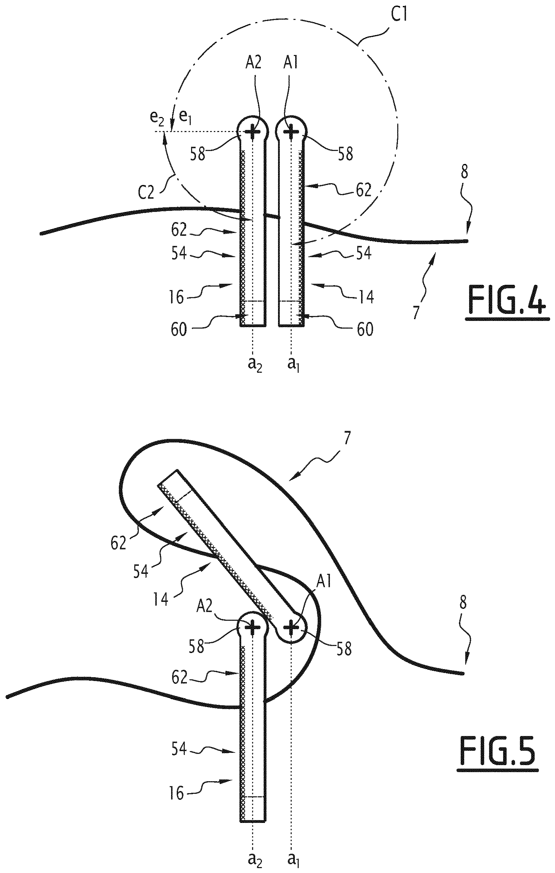

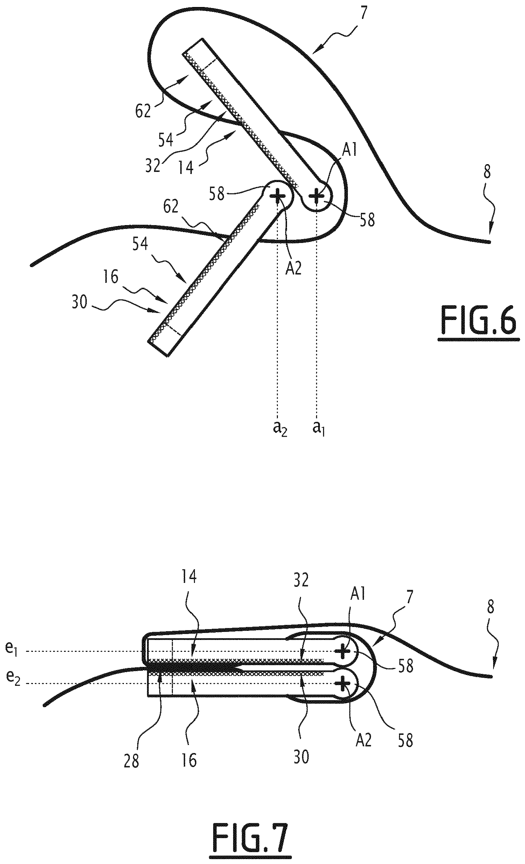

Le dispositif de traitement 12 est manœuvrable entre une configuration libre d'évolution des bras 14, 16, visible sur la figure 4 et une configuration de liaison des bras 14, 16 visible sur la figure 7. Dans la configuration libre, chaque bras 14, 16 est mobile en rotation autour de son axe de rotation respectif indépendamment de l'autre bras 14, 16. Dans la configuration de liaison, les bras 14, 16 sont solidaires en translation et en rotation.

En outre, le dispositif de traitement 12 comprend un moyen de maintien 28 manœuvrable entre une configuration de maintien dans laquelle le moyen de maintien 28 est propre à maintenir le dispositif 12 dans sa configuration de liaison et une configuration inactive. Le moyen de maintien 28 est par exemple constitué de deux organes de fixation complémentaires 30, 32, le moyen de maintien 28 étant dans la configuration de maintien lorsque les deux organes de fixation 30, 32 sont associés.

L'organe de guidage 25 permet de guider les bras 14, 16 transversalement l'un par rapport à l'autre. Cela signifie que la translation des bras 14 et 16 selon une direction perpendiculaire à la direction principale X est limitée par l'organe de guidage 25.

Un premier dispositif de traitement 12 est décrit en regard des figures 1 à 8.

Dans le premier mode de réalisation représenté sur la figure 2, le premier dispositif de traitement 12 comporte un corps allongé 26, un premier bras de pincement 14 et un deuxième bras de pincement 16 disposés dans le corps 26. L'organe de guidage 25 est formé par le corps 26 dans ce mode de réalisation.

En référence à la figure 2, le corps 26 est allongé selon un axe principal X entre une extrémité distale 34 et une extrémité proximale 36. L'axe principal X est colinéaire à l'axe longitudinal X du tuteur 18, lorsque le corps 26 est monté dans le tuteur 18.

Le corps 26 présente au moins une lumière centrale 38 s'étendant selon un axe de lumière parallèle à l'axe principal X. La lumière 38 débouche à la fois dans l'extrémité proximale 36 du corps 26 et dans l'extrémité distale 34 du corps 26.

Dans cet exemple, le corps 26 présente la forme d'un tube. Le corps 26 comporte une section sensiblement circulaire. Le diamètre du corps 26 est avantageusement compris entre 0,5 mm et 5 mm, notamment compris entre 0,5 mm et 1,5 mm, et de préférence égal à 1 mm. Il est formé par un matériau tel que du métal ou un polymère radio-opaque.

La longueur du corps 26 est inférieure à celle des bras 14, 16. Cette longueur est, par exemple, inférieure à 10 mm et est notamment comprise entre 4 mm et 8 mm.

Le corps 26 est avantageusement maintenu fixe en rotation par rapport à l'axe longitudinal X du tuteur 18. A cet effet, au moins une section transversale du corps 26 présente avantageusement une forme complémentaire à la section transversale intérieure du tuteur 18 au niveau de l'ouverture 24.

La lumière 38 est propre à recevoir au moins un bras 14, 16.

Dans cet exemple, le corps 26 présente une lumière 38 propre à recevoir les deux bras 14, 16. Le diamètre de la lumière 38 du corps 26 mesuré transversalement à l'axe de la lumière 38 est ainsi strictement supérieur à la somme du diamètre du premier bras 14 et du diamètre du deuxième bras 16 pour permettre la rotation de chaque bras 14, 16 dans la lumière 38 selon un axe de rotation A1, A2 colinéaire à l'axe de la lumière 38.

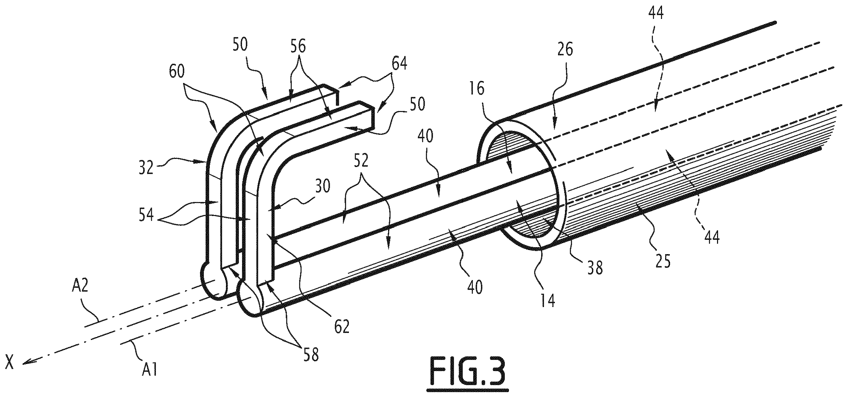

Comme cela est visible sur la figure 3, chaque bras 14, 16 présente une extrémité distale 40 de saisie de tissu faisant saillie par rapport au corps 26 et destinée à être diriger vers le prolapsus 7, une extrémité proximale 42 de manœuvre propre à coopérer avec un des mécanismes d'entrainements 20, 22 et opposée à l'extrémité distale de saisie 40 et une portion allongée 44 comprise entre l'extrémité distale de saisie 40 et l'extrémité proximale de manœuvre 42 et disposée dans une lumière 38 du corps 26.

Dans l'exemple, les portions allongées 44 des deux bras 14, 16 sont disposés dans la même lumière 38.

Dans le premier mode de réalisation, la portion allongée de chaque bras 14, 16 s'étend selon l'axe de rotation A1, A2 sensiblement parallèle à l'axe principal X. Ainsi, chaque bras 14, 16 est monté rotatif autour de son axe de rotation respectif A1, A2.

Dans le premier mode de réalisation, la longueur totale du bras 14, 16 mesurée selon l'axe de rotation A1, A2 entre l'extrémité distale de saisie 40 et l'extrémité proximale de manœuvre 42 est avantageusement comprise entre 10 mm et 20 mm. Avantageusement, un des bras 14, 16 est plus long que l'autre. Ceci facilite leur mise en mouvement indépendante.

Chaque bras 14, 16 comporte une section sensiblement circulaire. Le diamètre de la section du bras 14, 16 est, avantageusement, compris entre 0,2 mm et 1 mm. En variante, les portions 30 et 32 sont rectangulaires.

Les deux bras 14, 16 sont rigides.

Chaque bras 14, 16 est formé par un matériau tel que du métal, un polymère avec un marquage radio-opaque.

Sur la figure 2, l'extrémité proximale de manœuvre 42 de chaque bras 14, 16 fait saillie par rapport au corps 26 et s'étend dans le prolongement de la portion allongée 44.

L'extrémité proximale de manœuvre 42 de chaque bras 14, 16 comprend un module d'accroche 48.

Le module d'accroche 48 est propre à coopérer avec une partie du mécanisme d'entrainement 20, 22 comme cela sera décrit par la suite.

En référence à la figure 3, l'extrémité distale de saisie 40 de chaque bras 14, 16 comprend un crochet 50 propre à entrainer le prolapsus 7.

En outre, l'extrémité distale de saisie 40 de chaque bras 14, 16 porte un des organes de fixation 30, 32 complémentaires du moyen de maintien 28.

Chaque bras 14, 16 est, par exemple, monté libre en translation par coulissement le long de son axe de rotation A1, A2 à l'intérieur de la lumière 38 entre une position distale dans laquelle l'extrémité proximale de manœuvre 42 du bras 14, 16 est rapprochée de l'extrémité proximale 36 du corps 26 et une position proximale dans laquelle l'extrémité distale de saisie 40 du bras 14, 16 est rapprochée de l'extrémité distale 34 du corps 26.

Chaque bras 14, 16 est, en outre, monté dans le corps 26 libre en rotation entre une position angulaire d'accroche a1, a2 visible sur la figure 4 et une position angulaire d'enserrement e1, e2, visible sur la figure 7 autour d'un axe de rotation A1, A2 respectif. Les axes de rotations A1, A2 de chaque bras 14, 16 sont sensiblement parallèles.

Le vecteur normal de l'axe de rotation A1 est orienté depuis l'extrémité proximale de manœuvre 42 vers l'extrémité distale de saisie 40. Dans ce qui suit, le sens trigonométrique est définit par rapport à ce vecteur normal.

Le premier bras 14 est monté dans le corps 26 libre en rotation entre une position angulaire d'accroche a1 et une position angulaire d'enserrement e1 autour de l'axe de rotation A1.

La position angulaire d'accroche a1 du premier bras 14 et la position angulaire d'enserrement e1 du premier bras 14 délimitent dans le sens trigonométrique un premier secteur angulaire C1.

Le premier bras 14 est ainsi libre d'occuper une des positions angulaires intermédiaires situées dans le premier secteur angulaire C1, entre la position angulaire d'accroche a1 du premier bras 14 et la position angulaire d'enserrement e1 du premier bras 14.

Le premier bras 14 est représenté dans sa position angulaire d'accroche a1 sur la figure 4, dans une position intermédiaire sur la figure 5 et la figure 6 et dans sa position angulaire d'enserrement e1 sur la figure 7.

En outre, sur la figure 4, la position d'enserrement e1 du premier bras 14 et le premier secteur C1 sont représentés en pointillé.

Dans l'exemple représenté, l'angle mesuré dans le sens trigonométrique entre la position angulaire d'accroche a1 du premier bras 14 et la position angulaire d'enserrement e1 du premier bras 14 correspondant à l'étendue angulaire du secteur C1 est avantageusement sensiblement de 270°.

Le premier bras 14 est ainsi libre d'occuper une des positions angulaires intermédiaires formant avec la position angulaire d'accroche a1, un angle mesuré dans le sens trigonométrique inférieur à 270°.

Le deuxième bras 16 est monté dans le corps 26 libre en rotation entre une position angulaire d'accroche a2 et une position angulaire d'enserrement e2 autour d'un axe de rotation A2 sensiblement parallèle à l'axe principal X.

La position angulaire d'enserrement e2 du deuxième bras 16 et la position angulaire a2 d'accroche du deuxième bras 16 délimitent dans le sens trigonométrique un deuxième secteur angulaire C2.

Le deuxième bras 16 est ainsi libre d'occuper une des positions angulaires intermédiaires situées dans le deuxième secteur angulaire C2, entre la position angulaire d'enserrement e2 du deuxième bras 16 et la position angulaire d'accroche a2 du deuxième bras 16.

Le deuxième bras 16 est représenté dans sa position angulaire d'accroche a2 sur les figures 4 et 5, dans une position intermédiaire sur la figure 6 et dans sa position angulaire d'enserrement e2 sur la figure 7.

En outre, sur la figure 4, la position d'enserrement e2 du deuxième bras et le deuxième secteur C2 sont représentés en pointillé.

Dans l'exemple représenté, l'angle mesuré dans le sens trigonométrique entre la position angulaire d'enserrement e2 du deuxième bras 16 et la position angulaire d'accroche a2 du deuxième bras 16 correspondant à l'étendue angulaire du secteur C2 est sensiblement de 90°.

Le deuxième bras 16 est libre d'occuper des positions angulaires intermédiaires du deuxième bras 16 formant avec la position angulaire d'enserrement e2 du deuxième bras 16, un angle mesuré dans le sens trigonométrique inférieur à 90°.

Le premier secteur angulaire C1 et le deuxième secteur angulaire C2 sont avantageusement complémentaires. Cela signifie que toutes les positions angulaires par rapport à l'axe principal X sont accessibles par au moins un des bras 14, 16. La somme des angles des positions angulaires extrêmes des secteurs angulaires C1, C2 est supérieure ou égale à 360°.

Par exemple, lorsque les deux bras 14, 16 sont dans leur position angulaire d'accroches a1, a2 respectives, les crochets 50 sont parallèles. Lorsque les bras 14, 16 sont dans la position angulaire d'enserrement e1, e2 respectives, les crochets 50 sont parallèles.

En outre, chaque crochet 50 présente avantageusement une section rectangulaire afin de présenter une surface de contact propre à entrainer le prolapsus 7.

Chaque crochet 50 est sensiblement en forme de J, dans un plan longitudinal à l'axe de rotation A1, A2.

Le crochet 50 est avantageusement réalisé d'un seul tenant et est venu de matière avec le bras 14, 16. Ils sont par exemple réalisés à partir d'une matière métallique ou un polymère.

En référence à la figure 3, chaque crochet 50 comprend une tige 52, un siège 54 et un bec 56. Avantageusement la tige 52, le siège 54 et le bec 56 sont d'un seul tenant et fait d'une seule pièce.

La tige 52 s'étend selon l'axe de rotation A1, A2 du bras 14, 16 considéré et dans la continuité de la portion allongée 44.

Le siège 54 s'étend à partir de l'extrémité distale de la tige 52 sensiblement perpendiculairement à la tige 52.

Le siège 54 présente une extrémité de liaison 58 et une extrémité externe 60. L'extrémité de liaison 58 est liée à l'extrémité distale de la tige 52. L'extrémité externe 60 est liée au bec 56.

Le siège 54 de chaque crochet 50 présente une longueur adaptée au prolapsus 7 à traiter. Cette longueur est, par exemple, comprise entre 3 et 6 mm.

La section du siège 54 est sensiblement rectangulaire. Ainsi, chaque siège présente une surface externe orientée vers l'extrémité distale du dispositif 12, une surface interne opposée à la surface externe et orientée vers l'extrémité proximale du dispositif 12, une surface de repos et une surface de fixation 62 opposée à la surface de repos.

Lorsque les deux bras 14, 16 sont dans leur position angulaire d'accroche a1, a2, respective, l'angle, mesuré dans le sens trigonométrique par rapport au vecteur normal du premier bras 14, formé entre le siège 54 du premier bras 14 et le siège 54 du deuxième bras 16 est d'environ 360°.

Lorsque les deux bras 14, 16 sont dans leur position angulaire d'accroche a1, a2, comme illustré sur la figure 4, respective, les surfaces de repos de chaque bras 14, 16 sont en regard l'une de l'autre.

Lorsque les deux bras 14, 16 sont dans leur position angulaire d'enserrement respective e1, e2, comme illustré sur la figure 7, l'angle mesuré dans le sens trigonométrique par rapport au vecteur normal du premier bras 14 entre le siège 54 du premier bras 14 et le siège 54 du deuxième bras 16 mesuré est d'environ 0°.

Lorsque les deux bras 14, 16 sont dans leur position angulaire d'enserrement respective e1, e2, les surfaces de fixation 62 sont en regard l'une de l'autre.

Le moyen de maintien 28 propre à maintenir le dispositif 12 dans sa configuration de liaison est, dans cet exemple, porté par les surfaces de fixations 62 des sièges 54.

Chaque surface de fixation 62 comprend un organe complémentaire 30, 32. Lorsque chaque bras est dans sa position angulaire d'accroche a1, a2, les organes complémentaires 30, 32 de l'organe de maintien 28 sont dissociés. En effet, ils ne sont pas en contact.

Par exemple, chaque surface de fixation 62 comprend respectivement au moins une portion en saillie et au moins une portion en creux complémentaire.

Par exemple, le premier bras 14 de pincement comprend un siège 54 présentant un creux, et le deuxième bras 16 de pincement comporte un siège 54 comportant une portion en saillie complémentaire au creux.

En variante, le siège 54 du crochet 50 du premier bras 14 comporte une pluralité de dents et le siège 54 du crochet 50 du deuxième bras 16 comporte une pluralité de dents complémentaires aux dents du premier bras 14.

Le bec 56 s'étend à partir de l'extrémité externe 60 du siège 54.

Le bec 56 présente une extrémité distale et une extrémité proximale 64. L"extrémité distale du bec 56 est liée à l'extrémité externe 60 du siège 54. L'extrémité proximale 64 du bec 56 est libre.

L'extrémité proximale 64 du bec 56 est située transversalement à distance de la tige 52 de façon à permettre l'insertion du prolapsus 7 entre le bec 56 et la tige 52.

Avantageusement, le bec 56 s'étend sensiblement parallèlement à la tige 52 et sensiblement perpendiculairement au siège 54. La distance entre l'extrémité proximale 64 du bec 56 et la tige 52 est donc sensiblement la longueur du siège 54.

La longueur du bec 56 est avantageusement comprise entre 5 mm et 15 mm.

Le bec 56 présente avantageusement une surface rectangulaire d'entrainement du prolapsus 7.

Chaque mécanisme d'entrainement 20, 22 est propre à déplacer le bras 14, 16 qui lui est associé en rotation entre la position angulaire d'accroche a1, a2 et la position angulaire d'enserrement e1, e2.

Chaque mécanisme d'entrainement 20, 22 est par exemple monté sur le tuteur 18. De plus chaque mécanisme d'entrainement 20, 22 est fixé de manière amovible au module d'accroche 48 d'un bras 14, 16.

En outre, chaque mécanisme d'entrainement 20, 22 est propre à déplacer le bras 14, 16 qui lui est associé en translation entre la position distale et la position proximale.

En outre, chaque mécanisme d'entrainement 20, 22 est manipulable par un opérateur depuis une approche proximale.

Par exemple, le mécanisme d'entrainement 20, 22 comprend une tige allongée selon l'axe principale du bras 14, 16 associé et comprenant à son extrémité distale un module de commande 68. Le module de commande 68 présente une forme sensiblement complémentaire à la forme du module d'accroche 48.

Dans un exemple, le module d'accroche 48 présente une fente, et le module de commande 68 présente une languette de forme complémentaire à la fente.

Le fonctionnement du premier nécessaire de traitement 2 selon l'invention, lors du traitement d'un prolapsus 7 d'une valve 4 mitrale, va maintenant être décrit, en regard des figures 4 à 7.

Initialement, le premier nécessaire de traitement 2 est fourni. Le premier dispositif de traitement 12 est initialement dans la configuration libre. Le premier dispositif de traitement 12 est logé dans le tuteur 18. Le corps 26 est maintenu fixe en rotation par rapport à l'axe principal X du tuteur 18.

Les bras 14, 16 sont, par exemple, dans leur position angulaire d'accroche a1, a2. En outre, les bras 14, 16 sont dans leur position distale.

Le corps 26 est ainsi engagé dans le tuteur 18. Les bras 14, 16 font saillie axialement hors du tuteur 18.

Une gaine externe (non représentée) recouvre extérieurement le tuteur 18 et la partie du dispositif 12 qui fait saillie axialement hors du tuteur 18.

Ensuite, le nécessaire de traitement 2 est amené au niveau de la valve 4 mitrale à traiter, par exemple, par voie endovasculaire, c'est-à-dire par introduction dans la veine fémorale, montée jusqu'à l'oreillette droite, et passage dans l'oreillette gauche par voie transseptale.

La gaine externe est alors déplacée par rapport au tuteur 18 pour dévêtir la partie distale du dispositif 12 en saillie hors du tuteur 18 et faire apparaitre les extrémités distales 40 de saisie des bras 14, 16.

Le tuteur 18 est amené jusqu'à la localisation du prolapsus 7 sur le feuillet 8.

Comme illustré par la figure 4, le premier bras de pincement 14 et le deuxième bras de pincement 16 occupent alors leurs positions angulaires d'accroche a1, a2.

Le prolapsus 7 est alors saisi au niveau de son bord libre au moyen des crochets 50, en insérant le bord libre du prolapsus 7 entre la tige 52 et le bec 56 de chaque bras 14, 16 dans l'espace délimité distalement par le siège 54.

Plus particulièrement, la tige 52 de chaque crochet 50 est au-dessus de la surface supérieure du prolapsus 7, le bec 56 de chaque crochet 50 est sous la surface inférieure du prolapsus 7 et le siège 54 de chaque crochet 50 entre en contact avec le bord libre du prolapsus.

Par exemple, l'opérateur entraine chaque bras 14, 16 en translation vers sa position proximale au moyen du mécanisme d'entrainement 20, 22 associé.

L'opérateur positionne correctement le dispositif 12 puis à l'aide des mécanismes d'entrainement 20, 22 actionne indépendamment la rotation de chaque bras 14, 16.

L'opérateur déplace le premier bras 14 vers une position intermédiaire proche de sa position angulaire d'enserrement e1, comme illustré sur la figure 5. Par exemple, le premier bras 14 est tourné de 250° dans le sens trigonométrique. La rotation du premier bras 14 entraine la traction du bord libre du prolapsus 7 qui est entrainé par le siège 54 du premier bras 14. Le prolapsus 7 forme un premier repli dans lequel se loge le bec 56 du crochet 50 du premier bras 14, le premier repli fait saillie par rapport à deux régions latérales du prolapsus 7. Le corps 26 et les tiges 52 se logent dans la cavité définie entre le premier repli et une région latérale.

En outre, lors de ce déplacement, la tige 52 du premier bras 14 pivote autour de la tige 52 du deuxième bras 16.

Le tuteur 18 retient axialement le corps 26 et empêche sa rotation.

Puis, l'opérateur déplace le deuxième bras 16 vers une position intermédiaire proche de sa position angulaire d'enserrement e2, comme illustré sur la figure 6. Par exemple le deuxième bras 16 est tourné de 80° dans le sens anti-trigonométrique. La rotation du deuxième bras 16 entraine la traction du prolapsus 7 qui est entrainé par le siège 54 du deuxième bras 16.

L'opérateur exerce ensuite une rotation des deux bras 14, 16 au moyen des mécanismes d'entrainement 20, 22 afin de faire passer les bras 14, 16 dans leur position angulaire d'enserrement respectives e1 et e2.

Les surfaces de fixation 62 entrent ainsi en contact. Les organes de fixation complémentaires 30, 32 sont associés et le dispositif 12 passe dans la configuration de liaison.

Lorsque le prolapsus 7 est logé entre les deux bras 14, 16, le repli est écrasé contre la région latérale. Le prolapsus 7 présente alors une section transversale sensiblement en forme de S écrasé comme illustré sur la figure 7.

Le prolapsus 7 est maintenu entre les deux bras 14, 16 dans leur configuration de liaison.

Enfin, l'opérateur retire les mécanismes d'entrainement 20, 22 et le tuteur 18. Le dispositif 12 reste fixé au feuillet 8 en maintenant le prolapsus 7 replié.

La coaptation entre les bords libres des feuillets 6, 8 est rétablie lorsque la valve se ferme. Toutefois, le dispositif 12 est déplaçable conjointement avec le feuillet 8, indépendamment du feuillet 6, de sorte que la valve s'ouvre normalement, sans affecter la section de l'ouverture de passage du sang.

On conçoit ainsi que le nécessaire de traitement 2 selon l'invention traite un prolapsus 7 d'une valve mitrale 4 de façon particulièrement aisée et avec moins de risques pour le patient qu'une opération invasive.

En outre, le nécessaire de traitement 2 selon l'invention traite efficacement le prolapsus 7. En effet, une fois que le prolapsus 7 a été écrasé avec le dispositif 12 selon l'invention, la valve mitrale 4 peut se refermer de manière étanche et la coaptation des feuillets postérieur 8 et antérieur 6 de la valve 4 est assurée, tout en assurant une section de passage du sang la plus efficace possible lorsque la valve 4 est ouverte.

Le dispositif 12 selon l'invention s'applique par ailleurs plus généralement au traitement de prolapsus 7 sur d'autres tissus, tel que la valve aortique ou la valve tricuspide.

Le nécessaire 2 décrit sur ces figures est donc particulièrement simple et précis d'utilisation.

Dans une variante, chaque bras 14, 16 comprend un moyen de blocage en rotation. Le moyen de blocage en rotation du premier bras 14 est propre à empêcher le premier bras 14 d'occuper une position angulaire hors du premier secteur angulaire C1. Le moyen de blocage en rotation du deuxième bras 16 est propre à empêcher le deuxième bras 16 d'occuper une position angulaire hors du deuxième secteur angulaire C2.

Le moyen de blocage en rotation comprend, par exemple, une butée de rotation placée sur le bras 14, 16 considéré et une butée de rotation complémentaire placée dans la lumière 38 du corps 26.

En outre, le moyen de blocage en rotation est par exemple directionnel. Par exemple, le moyen de blocage en rotation autorise la rotation du bras 14, 16 considéré depuis sa position angulaire d'accroche a1, a2 vers sa position angulaire d'enserrement e1, e2 mais empêche la rotation du bras 14, 16 considéré depuis sa position angulaire d'enserrement e1, e2 vers sa position angulaire d'accroche a1, a2.

Ceci simplifie grandement le traitement du prolapsus 7 par le dispositif 12 grâce à une manipulation simple et guidée pour le chirurgien.

De préférence, l'extrémité proximale 42 de chaque bras 14, 16 comprend une butée de retenue. La butée de retenue est propre à empêcher la translation du bras 14, 16 considéré selon son axe de rotation A1, A2 au-delà de la position distale. Par exemple la butée de retenue est propre à collaborer avec une butée présente dans la lumière 38 du corps 26.

Par exemple, la butée de retenue du premier bras 14 est propre à empêcher la translation du premier bras 14 par rapport au corps 26 et la butée de retenue du deuxième bras 16 est propre à empêcher la translation du deuxième bras 16 par rapport au premier bras 14.

Dans une variante, le corps 26 comprend avantageusement deux lumières 38 coaxiale et chaque bras 14, 16 est disposé dans une des lumières 38. Avantageusement la distance entre les lumières 38 suivant une direction transversale à l'axe des lumières 38 au niveau de l'extrémité distale du corps 26 est comprise entre 0,3 mm et 3 mm.

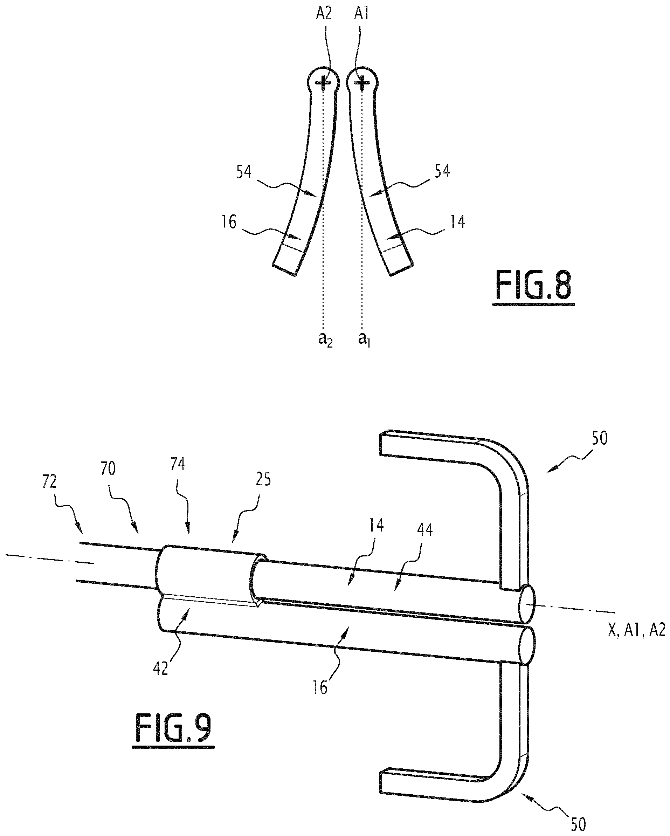

Dans une variante représentée sur la figure 8, le siège 54 du premier bras 14 forme un angle avec le siège 54 du deuxième bras 16 lorsque les deux bras 14, 16 sont dans leur position angulaire d'accroche a1, a2. Cet angle permet de solliciter élastiquement le dispositif 12, vers la configuration de liaison lorsque les deux bras 14, 16 sont proches de leur position angulaire d'enserrement e1, e2.

Un deuxième nécessaire de traitement 70 va à présent être décrit en référence à la figure 9.

Le deuxième nécessaire de traitement 70 comporte un deuxième dispositif de traitement 72 qui diffère du premier dispositif de traitement 12 en ce que les premier et deuxième axes de rotation A1 et A2 des bras 14, 16 sont confondus. Le deuxième nécessaire de traitement 70 diffère également du premier nécessaire de traitement 2 en ce que l'organe de guidage 25 est un anneau 74.

Dans le deuxième nécessaire de traitement 70, la portion allongée 44 du premier bras 14 s'étend selon le premier axe de rotation A1 sensiblement parallèle à l'axe principal X. La portion allongée 44 du deuxième bras 16 s'étend parallèlement au premier axe de rotation A1.

L'anneau 74 est solidaire du deuxième bras 16. Avantageusement, l'anneau 74 est d'une seule pièce et d'un seul tenant avec le deuxième bras 16. Par exemple, l'anneau est fixé à l'extrémité proximale 42 de manœuvre du deuxième bras 16. En variante, l'anneau 74 est fixé à la portion allongée 44 du deuxième bras 16.

L'anneau 74 définit une lumière s'étendant selon l'axe de rotation A1 du premier bras 14.

Le premier bras 14 traverse l'anneau 74. Le diamètre interne de l'anneau 74 est avantageusement légèrement supérieur au diamètre du premier bras 14 de sorte que l'anneau 74 et le deuxième bras 16 sont montés mobiles en rotation autour du premier bras 14. Ainsi, le deuxième bras 16 est monté rotatif autour de l'axe de rotation A1 du premier bras 14.

En variante ou en complément, une gorge est définie sur toute la circonférence du premier bras 14. La gorge forme une gouttière périphérique. L'anneau 74 est positionné dans la gorge. La gorge permet d'empêcher la translation de l'anneau 74 le long du premier bras 14. La dimension de la gorge selon l'axe X est légèrement supérieure à la dimension de l'anneau 74 selon l'axe X de sorte que les bords de la gorge n'empêchent pas la rotation de l'anneau 74 autour du premier bras 14.

Le deuxième bras 16 est ainsi monté mobile en rotation autour du premier bras 14 par l'intermédiaire de l'anneau 74.

En outre, le tuteur 18 est mobile en rotation autour de l'axe X. Le tuteur 18 est solidaire du deuxième bras 16. De la sorte, le tuteur 18 est le mécanisme d'entrainement 22 du deuxième bras 16. Le deuxième bras 16 est apte à être entrainé par le tuteur 18 qui est mobile en rotation. Le premier bras 14 est apte à être entrainé en rotation par le mécanisme d'entrainement 20.

Le fonctionnement du deuxième nécessaire de traitement 70, lors du traitement d'un prolapsus 7 d'une valve 4 mitrale, diffère du fonctionnement du premier nécessaire de traitement 4 en ce que la rotation du deuxième bras 16 est effectuée autour de l'axe de rotation du premier bras 14.

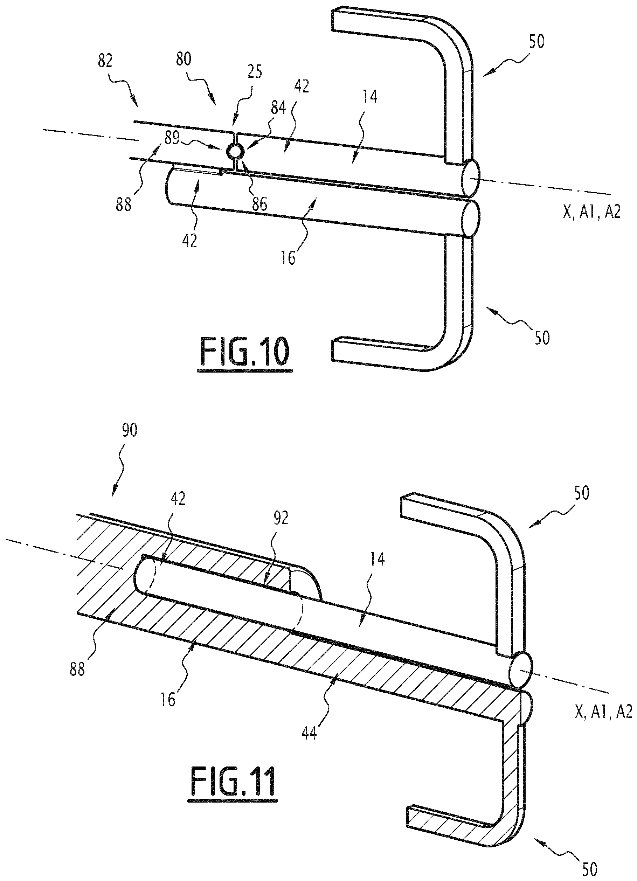

Un troisième nécessaire de traitement 80 va à présent être décrit en regard de la figure 10.

Le troisième nécessaire de traitement 80 comporte un troisième dispositif de traitement 82 qui diffère du premier dispositif de traitement 12 en ce que dans le troisième dispositif de traitement 82, les premier et deuxième axes de rotation A1 et A2 sont confondus. Le troisième nécessaire de traitement 80 diffère également du premier nécessaire de traitement 2 en ce que l'organe de guidage 25 est une rotule interne 84.

Dans le troisième nécessaire de traitement 80, le premier bras 14 et le deuxième bras 16 sont montés mobiles l'un par rapport à l'autre autour d'un même axe de rotation A1.

Le premier bras 14 comprend une extrémité proximale 42 apte à collaborer avec la rotule interne 84 et le deuxième bras 16. L'extrémité proximale 42 du premier bras 14 comporte un premier logement creux 86.

Le deuxième bras 16 comprend une extension cylindrique 88. L'extension cylindrique 88 s'étend dans le prolongement du premier bras 14. L'extension cylindrique 88 comporte un deuxième logement creux 89. L'extrémité proximale 42 du premier bras 14 et l'extension cylindrique 88 du deuxième bras 16 sont coaxiaux.

La rotule interne 84 est disposée entre les deux logements creux 86, 89.

La rotule interne 84 autorise la rotation du premier bras 14 sur le deuxième bras 16 mais empêche la translation du premier bras 14 par rapport au deuxième bras 16. En outre, la rotule interne 84 permet la rotation indépendante de chaque bras 14, 16 de l'axe de rotation A1. On entend par rotation indépendante que chaque bras 14, 16 est mobile autour de l'axe de rotation A1 sans entrainer la rotation de l'autre bras 14, 16.

Un quatrième dispositif 90 de traitement est décrit en regard de la figure 11. Le quatrième dispositif de traitement 90 est similaire au troisième dispositif 82 du troisième nécessaire de traitement 80, seules les différences étant détaillées ci-dessous.

L'extension cylindrique 88 du deuxième bras 16 comporte un logement creux 92 dans lequel l'extrémité proximale 42 du premier bras 14 est logée.

L'extrémité proximale 42 du premier bras 14 est mobile en rotation dans le logement creux 92 du deuxième bras 16 autour du premier axe de rotation A1.

L'organe de guidage 25 est ici formé par le logement 92 de l'extension 88 du deuxième bras 16.

Chaque bras 14, 16 est apte à tourner selon le premier axe de rotation A1.

En variante, l'organe de guidage 25 constitue une partie d'un mécanisme de maintien 28. Par exemple, l'extension 88 et l'extrémité proximale 42 comporte les éléments d'un mécanisme de verrouillage à baïonnette.

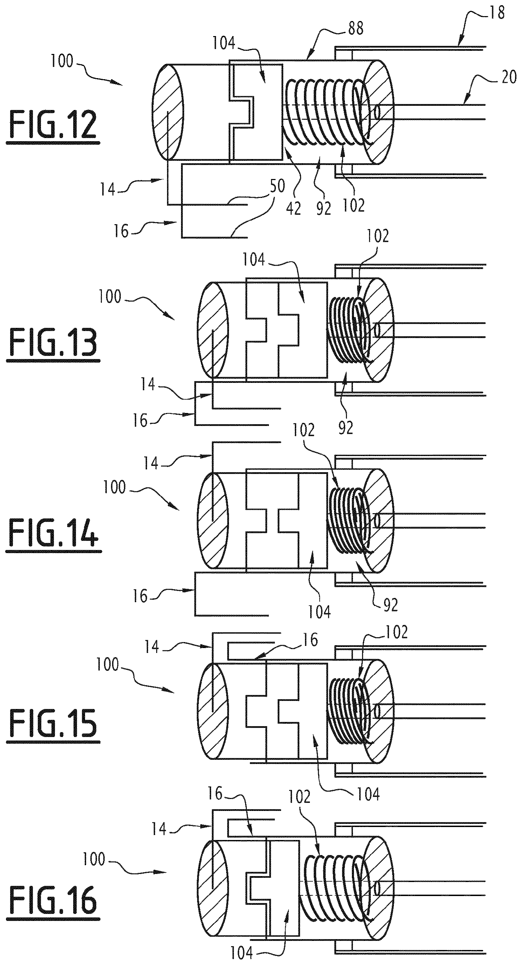

Un cinquième dispositif de traitement 100 va à présent être décrit en regard des figures 12 à 16. Le cinquième dispositif de traitement 100 est similaire au quatrième dispositif de traitement 90, seules les différences sont détaillées ici.

L'extension cylindrique 88 du deuxième bras 16 comporte un logement 92 dans lequel l'extrémité proximale 42 du premier bras 14, est apte à coulisser.

Le cinquième dispositif de traitement 100 comporte en outre un organe de rappel 102 logé entre l'extrémité proximale de l'extension cylindrique 88 du deuxième bras 16 et l'extrémité proximale du premier bras 14.

Le fond proximal de l'extension cylindrique 88 du deuxième bras 16 présente un bourrelet avantageusement circulaire permettant la retenue de l'organe de rappel 102.

La surface 104 de l'extrémité proximale 42 du premier bras 14 présente une forme complémentaire à la surface de l'extrémité distale de l'extension cylindrique 88 du deuxième bras 16.

Les formes complémentaires sont représentées emboitées sur les figures 12 et 16 et séparées sur les figures 13 à 15.

La surface 104 de l'extrémité proximale du premier bras 14 et l'extrémité distale de l'extension cylindrique 88 du deuxième bras 16 constituent un moyen de maintien du dispositif supplémentaire. Le moyen de maintien supplémentaire est manœuvrable entre une position initiale de verrouillage représentée sur la figure 12, des configurations inactives représentées sur les figures 13 à 15 et une configuration de maintien représentée sur la figure 16.

Lorsque les formes complémentaires sont emboitées, le deuxième bras 16 est solidaire en rotation du premier bras 14. Lorsque les formes complémentaires sont séparées, le premier bras 14 est mobile en rotation autour du premier axe de rotation A1, indépendamment du deuxième bras 16.

Le premier bras 14 est monté mobile en translation dans le logement creux 92 du deuxième bras 16 entre une première position déployée et une deuxième position rétractée.

L'organe de rappel 102 contraint le premier bras 14 vers sa position déployée. L'organe de rappel 102 est, par exemple, un ressort.

Le fonctionnement du cinquième dispositif 100 diffère du fonctionnement des dispositifs précédemment décrits en ce que le dispositif est fourni dans la configuration initiale de verrouillage.

Dans la configuration initiale de verrouillage représentée sur la figure 12, le ressort est déployé. Les formes complémentaires de la surface 104 de l'extrémité proximale du premier bras 14 et la surface de l'extrémité distale de l'extension cylindrique 88 du deuxième bras 16 sont emboitées. Les bras 14, 16 sont dans leur position angulaire d'accroche a1, a2. Le premier bras 14 est fixe en rotation autour de l'axe A2 du deuxième bras 16.

Après l'accroche du prolapsus 7, l'opérateur exerce une force entre le premier bras 14 et le deuxième bras 16 supérieure à la force de rappel de l'organe de rappel 102, de sorte que la surface 104 de l'extrémité proximale du premier bras 14 soit déplacée vers sa position rétractée. Le dispositif passe alors en configuration libre, les formes complémentaires sont séparées. Les bras 14, 16 sont indépendants en rotation.

Les bras 14, 16 sont pivotés comme cela a été précédemment décrit.

Sur la figure 14, le premier bras 14 a tourné, de sorte que la surface 104 de l'extrémité proximale du premier bras 14 et la surface de l'extrémité distale de l'extension cylindrique 88 du deuxième bras 16 ne sont pas emboitables. Ainsi, même si l'opérateur relâche la force exercée, le premier bras 14 reste libre en rotation par rapport au deuxième bras 16.

Lorsque les bras 14, 16 sont dans leurs positions respectives d'enserrement e1, e2, les formes complémentaires en regard sont dans une position angulaire relative permettant leur emboitement telle que représenté sur la figure 15.

A la fin de l'opération, telle que représentée sur la figure 16, l'opérateur n'exerce plus de force sur le ressort 102. Le premier bras 14 est déployé. Les formes complémentaires de la surface 104 de l'extrémité proximale du premier bras 14 et de la surface de l'extrémité distale de l'extension cylindrique 88 du deuxième bras s'emboitent de sorte à solidariser les bras 14, 16 en rotation.

Ceci permet un maintien efficace du dispositif. Avantageusement, le verrouillage n'est possible qu'en début ou en fin de rotation. Ceci permet d'empêcher une mise en place non complète du dispositif.

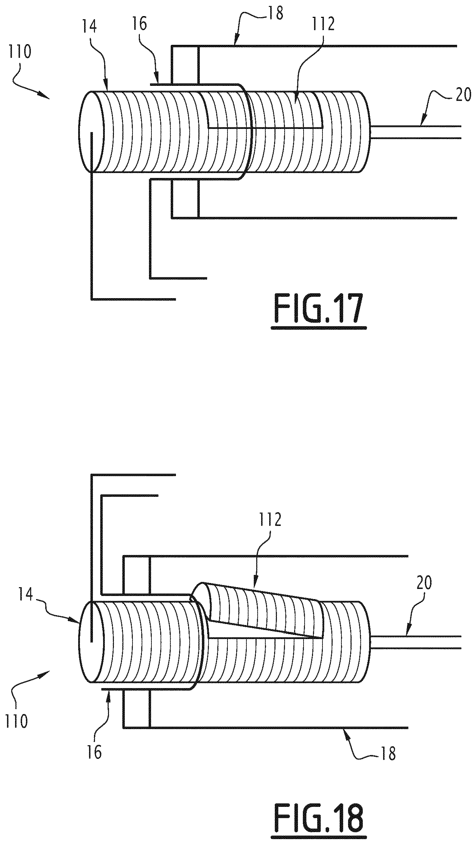

Un sixième dispositif de traitement 110 est représenté sur les figures 17 et 18.

Le sixième dispositif de traitement 110 est similaire au quatrième dispositif 90, seules les différences sont détaillées ici.

L'extension cylindrique 88 du deuxième bras 16 comporte un logement creux 92 dans lequel l'extrémité proximale 42 du premier bras 14 est logée. Le logement creux 92 débouche à la fois dans l'extrémité distale et dans l'extrémité proximale de l'extension cylindrique 88.

Avantageusement, l'extension cylindrique 88 présente une paroi intérieure filetée dans le logement creux 92. L'extrémité proximale 42 du premier bras 14 présente la forme d'un cylindre avec une paroi extérieure filetée avec un filetage complémentaire au filetage du logement 92. De la sorte, la rotation du premier bras 14 est liée à une translation par rapport au deuxième bras 16.

En outre, l'extrémité proximale 42 du premier bras 14 comporte une languette de butée 112.

La languette de butée 112 est, par exemple, une languette mobile transversalement entre une position rentrée et une position sortie.

Dans la position rentrée, la languette de butée 112 affleure la surface périphérique du cylindre.

Dans la position sortie, la languette de butée 112 fait saillie transversalement par rapport au premier bras 14. Dans la position sortie, l'extrémité distale de la languette de butée 112 est éloignée radialement de la surface périphérique du cylindre.

La languette de butée 112 est contrainte vers la position sortie.

Initialement, telle que représentée sur la figure 17, la languette de butée 112 est maintenue dans sa position rentrée par la paroi intérieure du logement 92 de l'extension cylindrique 88.

Lors de la mise en place du sixième dispositif 110, les bras 14, 16 tournent l'un par rapport à l'autre, de sorte que le premier bras 14 se déplace en translation le long de l'axe X par rapport au deuxième bras 16.

En configuration de liaison, la languette 112 sort du logement creux 92 de l'extension cylindrique 88.

La languette de butée 112 est libérée car elle n'est plus maintenue par la paroi interne du logement creux 92. La languette de butée 112 libérée se place en position sortie et empêche le retour du premier bras 14 et du deuxième bras 16 vers leurs positions initiales.

Le dispositif est donc sécurisé par la languette de butée 112. Dans une variante, le dispositif comporte plusieurs languettes.

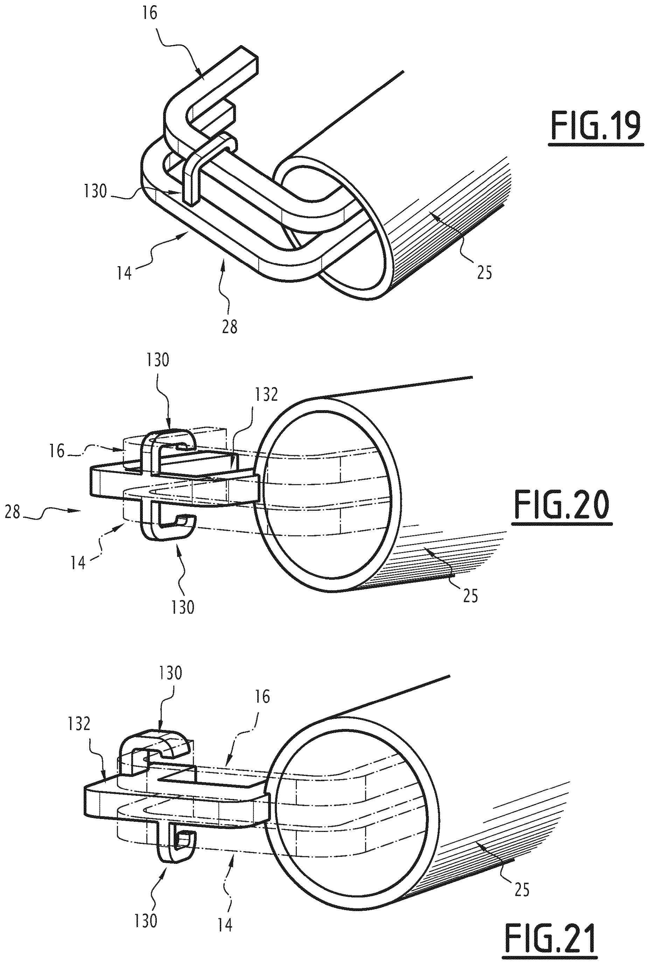

Des variantes de moyens de maintien 28 vont maintenant être décrits en regard des figures 19 à 21.

Ces moyens de maintien 28 peuvent être utilisés seuls ou en combinaison dans chacun des modes de réalisation du dispositif de traitement selon l'invention.

Tel que représenté sur la figure 19, le moyen de maintien 28 comprend un crochet de maintien 130. Le crochet de maintien 130 est fixé sur le siège 54 du premier bras 14.

Le crochet de maintien 130 est flexible et manœuvrable entre une position ouverte et une position fermée. Le crochet de maintien 130 est contraint vers sa position fermée. Le crochet de maintien 130 est, par exemple, réalisé dans une matière à mémoire de forme.

En outre, le crochet de maintien 130 est orienté perpendiculairement au siège 54 et à l'axe de rotation A1 du bras 14. Le crochet de maintien 130 définit un espace de capture du deuxième bras 16.

L'espace de capture présente une partie ouverte. La partie ouverte du crochet de maintien 130 est par exemple orientée vers l'opérateur. En variante, elle est orientée vers le cœur.

Lors de la mise en place du deuxième bras 16 après sa rotation, le siège 54 du crochet 50 du deuxième bras 16 pousse le crochet de maintien 130 vers sa position ouverte et passe dans l'espace de capture jusqu'à ce que le deuxième bras 16 soit dans sa position angulaire d'enserrement e2. Le crochet de maintien 130 revient alors à sa position fermée après le passage du deuxième bras 16. Le crochet de maintien 130 maintient alors le deuxième bras 16 dans sa position angulaire d'enserrement.

En variante, le crochet de maintien 130 est fixé sur le siège 54 du deuxième bras 16 et le crochet de maintien 130 permet la capture du premier bras 14.

Dans le cas où le crochet de maintien est fixé sur le premier bras 14, l'ouverture est avantageusement orientée vers l'opérateur.

Dans le cinquième dispositif, la fixation du crochet de maintien 130 au deuxième bras 16 est préférable.

Dans le mode de réalisation représenté sur la figure 20, une pièce additionnelle 132 est solidaire de l'organe de guidage 25.

La pièce additionnelle 132 présente la forme d'un U avec une branche interne solidaire du dispositif de guidage 25, une branche externe et un fond. La pièce additionnelle 132 est, par exemple, plate, en forme de plaquette.

La pièce complémentaire 132 comporte deux crochets de maintien 130. Les crochets de maintien 130 sont fixés sur le fond du U. Un crochet de maintien 130 supérieur est adapté pour fixer l'un des bras 14, 16 et un crochet de maintien inférieur 130 est adapté pour fixer l'autre des bras 14, 16. Chaque bras 14, 16 dans sa position angulaire d'enserrement e1, e2 est ainsi maintenu par le moyen de maintien 28.

Un autre moyen de maintien 28 est illustré sur la figure 21. Ce moyen de maintien 28 diffère du moyen de maintien 28 décrit dans la figure 20 en ce que les crochets de maintien 130 sont placés sur la branche externe de la pièce complémentaire 132 en regard de l'organe de guidage 25. En outre les crochets de maintien 28 présentent un espace de capture large.

L'espace de capture est adaptée pour la capture du tissu entre le bec du crochet 50 du bras et le crochet de maintien 130. Lorsque le bras est dans sa position de capture, le prolapsus est ainsi maintenu par la pièce en U par chaque crochet 50 des bras et par le crochet de maintien 130. Chaque crochet de maintien maintient ainsi à la fois un bras 14, 16 et le prolapsus 7.

De tels moyens de maintien 28 assurent une tenue sure et à long terme du dispositif de traitement 12, 72, 82, 90, 100, 110.

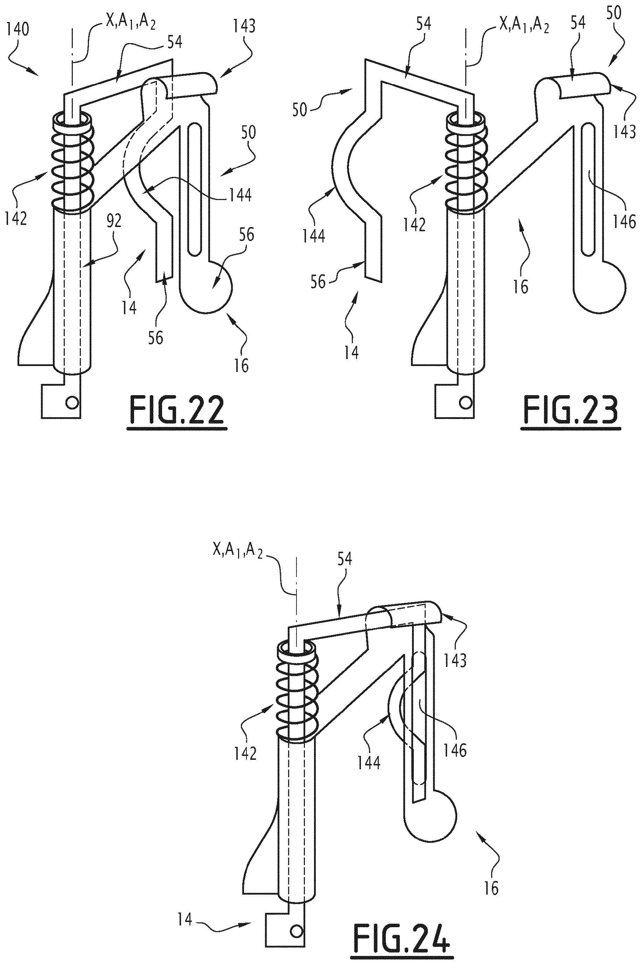

Un septième dispositif de traitement 140 est représenté sur les figures 22, 23 et 24. Le septième dispositif de traitement 140 est similaire au quatrième dispositif de traitement 90 décrit en regard de la figure 11. Seules les différences sont détaillées ci-dessous.

Dans ce dispositif, chaque bras 14, 16 est propre à tourner selon le premier axe de rotation A1. Le premier bras 14 et le deuxième bras 16 sont montés mobiles en translation l'un par rapport à l'autre par coulissement du premier bras 14 dans le logement creux 92 du deuxième bras 16 entre une première position totalement déployée, illustrée sur les figures 22 et 23, une position de verrouillage, visible sur la figure 24, et une deuxième position rétractée de mise en place.

Le septième dispositif de traitement 140 comporte un organe de rappel 142. L'organe de rappel 142 contraint le premier bras 14 vers la première position totalement déployée et vers la position de verrouillage. L'organe de rappel 142 est par exemple disposé comme l'organe de rappel 102 du cinquième dispositif de traitement 100. L'organe de rappel 142 est, par exemple, un ressort.

Le crochet 50 du deuxième bras 16 comporte un rebord de maintien 143. Dans l'exemple illustré sur les figures 22, 23 et 24, le rebord de maintien 143 est une rigole. Le rebord de maintien 143 est adapté pour bloquer le siège 54 du premier bras 14 dans la configuration de liaison des bras 14, 16, illustrée par la figure 24.

Le bec 56 du premier bras 14 présente un décrochement 144 en saillie. Le décrochement 144 fait saillie par rapport à la direction du bec selon une direction transversale à l'axe A1. Le décrochement présente une forme sensiblement incurvée, par exemple une forme de C. Le bec 56 du deuxième bras 16 présente une fente 146 complémentaire au décrochement 144 du premier bras 14. La fente 146 est adaptée pour que le décrochement 144 de forme incurvée s'engage dans la fente 146 de sorte à bloquer les bras 14, 16 l'un par rapport à l'autre, lorsque les bras 14, 16 sont en configuration de liaison.

Lors de la mise en place du dispositif 140, l'opérateur déplace le premier bras 14 vers sa position rétractée de mise en place à l'encontre de la compression de l'organe de rappel 142, avant d'arriver en position d'enserrement. Lorsque l'utilisateur relâche le bras 14, l'organe de rappel 142 pousse le siège 54 du premier bras 14 dans le rebord de maintien 143 du deuxième bras 16 vers la position de verrouillage. Le rebord de maintien 143 bloque ainsi le siège 54 qui est sollicité contre le rebord par l'organe de rappel 142. Simultanément au cours de la rotation, le décrochement 144 de forme incurvée du bec 56 du premier bras 14 entre dans la fente 146. La forme de ce décrochement 144 est avantageuse pour pouvoir faciliter la rotation du premier bras 14 en même temps que la translation du premier bras 14 vers la position déployée de verrouillage entraînée par l'organe de rappel 142.

Lorsque les bras 14, 16 sont en configuration de liaison, une partie du tissu entraîné par le bec 56 du premier bras 14 est bloquée dans la fente 146. Le tissu est ainsi maintenu à la fois par le siège 54 et par le bec 56 de chaque crochet 50.

La fente 146 permet un écrasement du tissu entre le bec 56 de chaque bras 14, 16. L'organe de rappel 142 permet la translation vers la configuration rétractée du premier bras 14 par rapport au deuxième bras 16 pour pouvoir bloquer le siège dans le rebord de maintien 143. L'organe de rappel 142 empêche le déblocage intempestif d'un bras 14, 16 par rapport à l'autre.

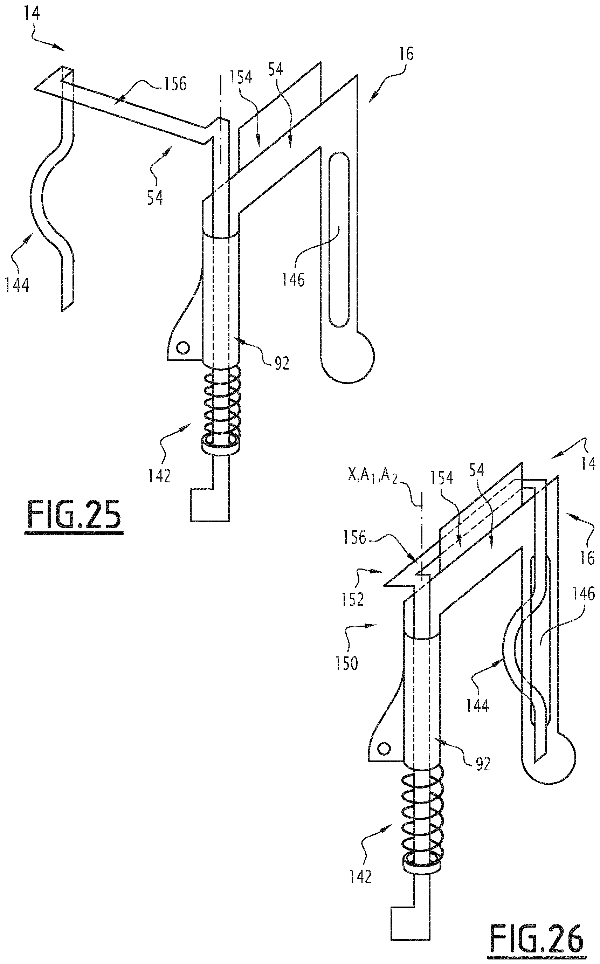

Un huitième dispositif de traitement 150 est représenté sur les figures 25 et 26. L'organe de maintien 28 entre le premier bras et le deuxième bras comprend un dispositif d'encliquetage 152. Le dispositif d'encliquetage comprend une languette 154 et un clip 156 propre à coopérer avec la languette 154.

La languette 154 s'étend en saillie à partir du siège 54 du deuxième bras 16 du côté de l'extrémité distale du bras 14. Dans l'exemple de la figure 25, la languette 154 est perpendiculaire à la direction du siège 54, en variante, elle s'étend dans un plan oblique.

Le clip 156 est positionné sur le siège 54 du premier bras 14. Le clip 156 présente un logement propre à recevoir la languette 154.

Lors de la mise en place du huitième dispositif 150, l'opérateur déplace le deuxième bras 16 en comprimant le ressort 142 vers une position éloignée du premier bras 14. Le premier bras 14 est alors dans la position de mise en place par rapport au deuxième bras 16. L'opérateur effectue ensuite une rotation du premier bras 14 par rapport au deuxième bras 16 jusqu'à la position angulaire d'enserrement. Puis, lorsque l'opérateur relâche le bras 16, l'organe de rappel 142 ramène le deuxième bras 16 dans la position de verrouillage, ce qui pousse la languette du deuxième bras 16 dans le logement du clip 156 du premier bras 14.

Lorsque les deux bras 14, 16 sont en position de verrouillage, représentée sur la figure 26, la languette 154 est encliquetée dans le clip 156 du premier bras 14.

Les dispositifs de traitement 12, 72, 82, 90, 100, 110, 140, 150 précédemment décrits sont délivrables. Ils peuvent être largués et laissés en place dans l'organisme. Du fait des moyens de maintien 28, ils restent en position après l'intervention de l'opérateur.

Lorsque les bras 14, 16 sont en configuration de liaison, une partie du tissu entraîné par le bec 56 du premier bras 14 est bloquée dans la fente 146. Le tissu est ainsi maintenu à la fois par le siège 54 et par le bec 56 de chaque crochet 50.

La fente 146 permet un écrasement du tissu entre le bec 56 de chaque bras 14, 16. L'organe de rappel 142 permet la translation vers la configuration rétractée du premier bras 14 par rapport au deuxième bras 16 pour pouvoir bloquer le siège dans le rebord de maintien 143. L'organe de rappel 142 empêche le déblocage intempestif d'un bras 14, 16 par rapport à l'autre.

Un huitième dispositif de traitement 150 est représenté sur les figures 25 et 26. L'organe de maintien 28 entre le premier bras et le deuxième bras comprend un dispositif d'encliquetage 152. Le dispositif d'encliquetage comprend une languette 154 et un clip 156 propre à coopérer avec la languette 154.

La languette 154 s'étend en saillie à partir du siège 54 du deuxième bras 16 du côté de l'extrémité distale du bras 14. Dans l'exemple de la figure 25, la languette 154 est perpendiculaire à la direction du siège 54, en variante, elle s'étend dans un plan oblique.

Le clip 156 est positionné sur le siège 54 du premier bras 14. Le clip 156 présente un logement propre à recevoir la languette 154.

Lors de la mise en place du huitième dispositif 150, l'opérateur déplace le deuxième bras 16 en comprimant le ressort 142 vers une position éloignée du premier bras 14. Le premier bras 14 est alors dans la position de mise en place par rapport au deuxième bras 16. L'opérateur effectue ensuite une rotation du premier bras 14 par rapport au deuxième bras 16 jusqu'à la position angulaire d'enserrement. Puis, lorsque l'opérateur relâche le bras 16, l'organe de rappel 142 ramène le deuxième bras 16 dans la position de verrouillage, ce qui pousse la languette du deuxième bras 16 dans le logement du clip 156 du premier bras 14.

Lorsque les deux bras 14, 16 sont en position de verrouillage, représentée sur la figure 26, la languette 154 est encliquetée dans le clip 156 du premier bras 14.

Les dispositifs de traitement 12, 72, 82, 90, 100, 110, 140, 150 précédemment décrits sont délivrables. Ils peuvent être largués et laissés en place dans l'organisme. Du fait des moyens de maintien 28, ils restent en position après l'intervention de l'opérateur.

## Revendications

1. Dispositif (12, 72, 82, 90, 100, 110, 140, 150) de traitement d'un tissu corporel présentant un prolapsus (7), comprenant :
- au moins deux bras (14, 16), chaque bras (14, 16) présentant une extrémité distale (40) de saisie du tissu, une extrémité proximale de manœuvre (42) opposée à l'extrémité distale de saisie (40) et une portion allongée (44) comprise entre l'extrémité distale de saisie (40) et l'extrémité proximale de manœuvre (42), chaque portion allongée (44) s'étendant selon une direction principale X, chaque bras (14, 16) étant mobile en rotation entre une position angulaire d'accroche (a1, a2) et une position angulaire d'enserrement (e1, e2) autour d'un axe de rotation (A1, A2) sensiblement parallèle à la direction principale (X), le dispositif (12, 72, 82, 90, 100, 110, 140, 150) comprenant un organe de guidage (25) apte à limiter le déplacement transversal des bras (14, 16) l'un par rapport à l'autre selon une direction perpendiculaire à la direction principale (X),
caractérisé en ce le dispositif (12) est manœuvrable entre une configuration libre, dans laquelle chaque bras (14, 16) est mobile en rotation indépendamment de l'autre bras (14, 16) et une configuration de liaison dans laquelle les deux bras (14, 16) sont solidaires en rotation,
en que le dispositif (12, 72, 82, 90, 100, 110, 140, 150) est configuré pour être largué et laissé en place dans l'organisme, et
en ce que l'organe de guidage comporte un corps (26) allongé suivant la direction principale (X), le corps (26) présentant au moins une lumière (38) recevant chaque bras (14, 16), les deux bras (14, 16) étant avantageusement disposés dans la même lumière (38) ou en ce que les bras (14, 16) sont montés rotatifs autour d'un même axe, un des bras (16) étant monté rotatif sur ou dans un autre bras (14).

2. Dispositif (12, 72, 82, 90, 100, 110, 140, 150) de traitement selon la revendication 1, dans lequel le dispositif (12) comprend un moyen de maintien (28) propre à maintenir le dispositif (12, 72, 82, 90, 100, 110, 140, 150) dans la configuration de liaison.

3. Dispositif (12, 72, 82, 90, 100, 110, 140, 150) de traitement selon l'une quelconque des revendications 1 à 2, dans lequel chaque extrémité distale de saisie (40) comprend un crochet (50), propre à entrainer le tissu.

4. Dispositif (12, 72, 82, 90, 100, 110, 140, 150) de traitement selon l'une quelconque des revendications 1 à 3, comprenant une butée de retenue propre à empêcher la translation d'au moins un bras (14, 16) selon la direction principale (X) vers l'extrémité distale de saisie (40).

5. Dispositif (12, 72, 82, 90, 100, 110, 140, 150) de traitement selon l'une quelconque des revendications 1 à 4, dans lequel la rotation du premier bras (14) est libre dans un premier secteur angulaire (C1) autour d'un axe parallèle à la direction principale (X) et la rotation du deuxième bras (16) est libre dans un deuxième secteur angulaire (C2), le deuxième secteur angulaire (C2) étant complémentaire du premier secteur angulaire (C1).

6. Dispositif (12, 72, 82, 90, 100, 110, 140, 150) de traitement selon l'une quelconque des revendications 1 à 5, dans lequel le premier bras (14) et le deuxième bras (16) sont montés mobiles en translation l'un par rapport à l'autre entre une position de verrouillage et une position de mise en place, le dispositif (12, 72, 82, 90, 100, 110, 140, 150) comprenant un organe de rappel (102, 142) propre à contraindre le premier bras (14) et/ou le deuxième bras (16) vers la position de verrouillage.

7. Nécessaire de traitement (2, 70, 80) d'un tissu corporel présentant un prolapsus (7), comprenant :
- un dispositif (12, 72, 82, 90, 100, 110, 140, 150) de traitement selon l'une quelconque des revendications 1 à 6, et
- au moins deux mécanismes d'entrainement (20, 22), chaque mécanisme d'entrainement (20, 22) étant propre à déplacer un bras (14, 16) distinct en rotation entre la position angulaire d'accroche (a1, a2) et la position angulaire d'enserrement (e1, e2).

8. Nécessaire de traitement (2, 70, 80) selon la revendication 7 comprenant en outre un tuteur (18), s'étendant suivant un axe longitudinal sensiblement parallèle à la direction principale (X) et définissant au moins une ouverture (24) de retenue du dispositif (12, 72, 82, 90, 100, 110, 140, 150).

## Patentansprüche

1. Vorrichtung (12, 72, 82, 90, 100, 110, 140, 150) zur Behandlung eines Körpergewebes, das einen Prolaps (7) aufweist, umfassend:
- mindestens zwei Arme (14, 16), wobei jeder Arm (14, 16) ein distales Greifende (40) des Gewebes, ein proximales Manövrierende (42) gegenüber dem distalen Greifende (40) und einen länglichen Abschnitt (44) zwischen dem distalen Greifende (40) und dem proximalen Manövrierende (42) aufweist, wobei sich jeder längliche Abschnitt (44) gemäß einer Hauptrichtung X erstreckt, wobei jeder Arm (14, 16) zwischen einer Winkelhakenposition (a1, a2) und einer Winkeleinspannposition (e1, e2) um eine Rotationsachse (A1, A2), die etwa parallel zur Hauptrichtung (X) ist, rotatorisch beweglich ist, wobei die Vorrichtung (12, 72, 82, 90, 100, 110, 140, 150) ein Führungsorgan (25) umfasst, das imstande ist, die transversale Verlagerung der Arme (14, 16) zueinander gemäß einer Richtung, die senkrecht zu der Hauptrichtung (X) ist, zu begrenzen,
**dadurch gekennzeichnet, dass** diese Vorrichtung (12) zwischen einer freien Konfiguration, in welcher jeder Arm (14, 16) unabhängig von dem anderen Arm (14, 16) rotatorisch beweglich ist, und einer Verbindungskonfiguration, in welcher die zwei Arme (14, 16) rotatorisch fest verbunden sind, manövrierbar ist,
dass die Vorrichtung (12, 72, 82, 90, 100, 110, 140, 150) konfiguriert ist, um losgelassen zu werden und vor Ort im Organismus zu verbleiben, und
und dass das Führungsorgan einen länglichen Körper (26) gemäß der Hauptrichtung (X) aufweist, wobei der Körper (26) mindestens eine Öffnung (38) aufweist, die jeden Arm (14, 16) aufnimmt, wobei die zwei Arme (14, 16) in vorteilhafter Weise in derselben Öffnung (38) angeordnet sind oder dass die Arme (14, 16) rotatorisch um eine selbe Achse angebracht sind, wobei einer der Arme (16) auf oder in dem anderen Arm (14) rotatorisch angebracht ist.

2. Behandlungsvorrichtung (12, 72, 82, 90, 100, 110, 140, 150) nach Anspruch 1, wobei die Vorrichtung (12) ein Haltemittel (28) umfasst, das imstande ist, die Vorrichtung (12, 72, 82, 90, 100, 110, 140, 150) in der Verbindungskonfiguration zu halten.

3. Behandlungsvorrichtung (12, 72, 82, 90, 100, 110, 140, 150) nach einem der Ansprüche 1 bis 2, wobei jedes distale Greifende (40) einen Haken (50) umfasst, der imstande ist, das Gewebe mitzunehmen.

4. Behandlungsvorrichtung (12, 72, 82, 90, 100, 110, 140, 150) nach einem der Ansprüche 1 bis 3, umfassend einen Halteanschlag, der imstande ist, die Translation von mindestens einem Arm (14, 16) gemäß der Hauptrichtung (X) in Richtung des distalen Greifendes (40) zu verhindern.

5. Behandlungsvorrichtung (12, 72, 82, 90, 100, 110, 140, 150) nach einem der Ansprüche 1 bis 4, wobei die Rotation des ersten Arms (14) frei ist in einem ersten Winkelsektor (C1) um eine Achse, die parallel zur Hauptrichtung (X) ist, und die Rotation des zweiten Arms (16) frei ist in einem zweiten Winkelsektor (C2), wobei der zweite Winkelsektor (C2) komplementär zum ersten Winkelsektor (C1) ist.

6. Behandlungsvorrichtung (12, 72, 82, 90, 100, 110, 140, 150) nach einem der Ansprüche 1 bis 5, wobei der erste Arm (14) und der zweite Arm (16) zwischen einer Verriegelungsposition und einer Platzierungsposition zueinander translatorisch beweglich angebracht sind, wobei die Vorrichtung (12, 72, 82, 90, 100, 110, 140, 150) ein Rückholorgan (102, 142) umfasst, das imstande ist, den ersten Arm (14) und/oder den zweiten Arm (16) in die Verriegelungsposition zu zwingen.

7. Behandlungsset (2, 70, 80) eines Körpergewebes, das einen Prolaps (7) aufweist, umfassend:
- eine Behandlungsvorrichtung (12, 72, 82, 90, 100, 110, 140, 150) nach einem der Ansprüche 1 bis 6, und
- mindestens zwei Antriebsmechanismen (20, 22), wobei jeder Antriebsmechanismus (20, 22) imstande ist, einen bestimmten Arm (14, 16) zwischen der Winkelhakenposition (a1, a2) und der Winkelspannposition (e1, e2) rotierend zu verlagern.

8. Behandlungsset (2, 70, 80) nach Anspruch 7, umfassend ferner eine Stütze (18), die sich gemäß einer zur Hauptrichtung (X) etwa parallelen Längsachse erstreckt und mindestens eine Halteöffnung (24) der Vorrichtung (12, 72, 82, 90, 100, 110, 140, 150) definiert.

## Claims

1. A treatment device (12, 72, 82, 90, 100, 110, 140, 150) for treating a body tissue presenting a prolapse (7), comprising:
- at least two arms (14, 16), each arm (14, 16) having a distal end (40) for gripping the tissue, a proximal maneuvering end (42) opposite the distal gripping end (40), and an elongate portion (44) comprised between the distal gripping end (40) and the proximal maneuvering end (42), each elongate portion (44) extending along a main direction X,
each arm (14, 16) being rotatable between an angular catching position (a1, a2) and an angular clamping position (e1, e2) around a rotation axis (A1, A2) substantially parallel to the main direction (X), the device (12, 72, 82, 90, 100, 110, 140, 150) comprising a guide member (25) able to limit the transverse movement of the arms (14, 16) relative to one another along a direction perpendicular to the main direction (X)
**characterized in that** the device (12) is maneuverable between a free configuration, in which each arm (14, 16) is rotatable independently of the other arm (14, 16), and a connecting configuration, in which the two arms (14, 16) are secured in rotation,
**in that** the device (12, 72, 82, 90, 100, 110, 140, 150) is configured to be released and left in place in the body;
**in that** the guide member includes an elongate body (26) along the main direction (X), the body (26) having at least one opening (38) receiving each arm (14, 16), the two arms (14, 16) advantageously being positioned in the same opening (38), or **in that**
the arms (14, 16) are mounted rotating around a same axis, one of the arms (16) being mounted rotating on or in another arm (14).

2. The treatment device (12, 72, 82, 90, 100, 110, 140, 150) according to claim 1, wherein the device (12) comprises a maintaining system (28) able to keep the device (12, 72, 82, 90, 100, 110, 140, 150) in the connecting configuration.

3. The treatment device (12, 72, 82, 90, 100, 110, 140, 150) according to any one of claims 1 to 2, wherein each distal gripping end (40) comprises a hook (50) able to draw the tissue.

4. The treatment device (12, 72, 82, 90, 100, 110, 140, 150) according to any one of claims 1 to 3, comprising a retaining stop able to prevent the translation of at least one arm (14, 16) in the main direction (X) toward the distal gripping end (40).

5. The treatment device (12, 72, 82, 90, 100, 110, 140, 150) according to any one of claims 1 to 4, wherein the rotation of the first arm (14) is free in a first angular sector (C1) around an axis parallel to the main direction (X) and the rotation of the second arm (16) is free in a second angular sector (C2), the second angular sector (C2) being complementary to the first angular sector (C1).

6. The treatment device (12, 72, 82, 90, 100, 110, 140, 150) according to any one of claims 1 to 5, wherein the first arm (14) and the second arm (16) are mounted translatably relative to one another between a locking position and a placement position, the device (12, 72, 82, 90, 100, 110, 140, 150) comprising a return member (102, 142) able to stress the first arm (14) and/or the second arm (16) toward the locking position.

7. A treatment kit (2, 70, 80) for treating a body tissue presenting a prolapse (7), comprising:
- a treatment device (12, 72, 82, 90, 100, 110, 140, 150) according to any one of claims 1 to 6, and
- at least two drawing mechanisms (20, 22), each drawing mechanism (20, 22) being able to rotate a separate arm (14, 16) between the angular catching position (a1, a2) and the angular clamping position (e1, e2).

8. The treatment kit (2, 70, 80) according to claim 7, further comprising a stay (18), extending along a longitudinal axis substantially parallel to the main direction (X) and defining at least one retaining opening (24) of the device (12, 72, 82, 90, 100, 110, 140, 150).
